# EUROPEAN PATENT APPLICATION

(11) **EP 1 914 224 A1**
(43) Date of publication of application: **23.04.2008**
(21) Application number: 06122584.3
(22) Date of filing: 19.10.2006
(51) Int. Cl.: C07C 271/30, A61K 31/137

(54) **Amorphous organic compound**

(71) Applicant: SANDOZ AG, 4056 Basel (CH)
(72) Inventor: The designation of the inventor has not yet been filed
(74) Representative: Fauss-Berghus, Waltraud

(57) **Abstract**

The present invention relates to stable amorphous Cinacalcet hydrochloride, methods for the preparation thereof and to pharmaceutical compositions comprising stable amorphous Cinacalcet hydrochloride.

## Description

The present invention relates to stable amorphous Cinacalcet hydrochloride, methods for the preparation thereof, the use of stable amorphous Cinacalcet hydrochloride in the treatment of hyperparathyroidism and of hypercalcemia and to pharmaceutical compositions comprising stable amorphous Cinacalcet hydrochloride.

Cinacalcet hydrochloride, N-[1-(R)-(-)-(1-naphthyl)ethyl]-3-[3-(trifluoromethyl)phenyl]-1-aminopropane hydrochloride, shown as Compound (I) below

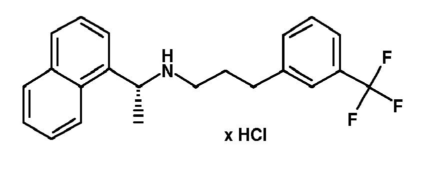

### Compound (I)

is a novel second generation calcimimetic that modulates the extra cellular calcium sensing receptor (CaR) by making it more sensitive to the calcium suppressive effects on parathyroid hormone (PTH). It is used in a treatment for primary and secondary hyperparathyroidism. Hyperparathyroidism is characterized by high levels of circulating calcium due to an increased secretion of parathyroid hormone by one or more of the parathyroid glands. Hyperparathyroidism can lead to e.g. osteoporosis; patients with renal failure suffering from secondary hyperparathyroidism have for example an increased risked of renal bone disease, soft-tissue calcifications and vascular disease.

The preparation of Cinacalcet is, for example, described in Drugs of the Future 2002, 27(9), 831-836 and its use in the treatment of primary and secondary hyperparathyroidism has been the subject of several research articles, e.g. Expert opinion on investigational drugs (2003), 12(8), 1413-21.

Cinacalcet is sold e.g. in US as Sensipar® in the form of tablets. Sensipar® is to be used in the treatment of hyperparathyroidism and of hypercalcemia.

The isolation of Cinacalcet as hydrochloride is not described in the patent literature. US 6211244 examplifies the synthesis and isolation of analogues. Hydrochlorides of these analogues are prepared by the precipitation using HCl g in ether or hexane in combination with HCl g in ether. This method is not applicable to large scale synthesis.

Amorphous products often show improved absorption in humans, in particular the amorphous form alone, a dispersion of the drug in a matrix material, or the drug absorbed onto a substrate may dissolve more rapidly than the crystalline form of the drug, often dissolving faster than the drug can precipitate from the solution. As a result, the amorphous form may show an increasing bioavailability.

However, amorphous products often show a chemical stability which renders them unsuitable for the preparation of medicaments.

There is thus a need for a stable amorphous form of cinacalcet hydrochloride which is suitable for application in pharmaceutical compositions.

### Summary of the invention

The present invention provides stable amorphous Cinacalcet Hydrochloride (Compound I), methods for the preparation thereof, and pharmaceutical compositions comprising stable amorphous Cinacalcet Hydrochloride.

### Detailed description of the invention

The present inventors have surprisingly identified an amorphous form of Cinacalcet Hydrochloride which is stable upon storage. This property is important and proves advantageous for the desired use of Cinacalcet Hydrochloride in pharmaceutical formulations. Moreover, the amorphous form of Cinacalcet Hydrochloride of the invention lends itself to facile manufacturing of pharmaceutical compositions.

The invention therefore relates to a stable amorphous form of Cinacalcet Hydrochloride. By "stable" it is meant that the amorphous form of Cinacalcet Hydrochloride of the invention shows very little degradation upon storage under stress conditions, i.e. there is essentially no decrease in assay of Cinacalcet as measured by HPLC, the measurement being detailed in example 1, the decrease being less than 0.3 area % when stored at 60°C for 24 hours.

In a preferred embodiment, the stable amorphous form of Cinacalcet Hydrochloride exhibits an increase in impurity levels as measured by HPLC as described above of less than 0.2 area % when stored at 25°C/60% relative humidity for one month, in particular even after storage for 6 months.

The invention further relates to processes for the production of the stable amorphous form of Cinacalcet Hydrochloride of the invention starting from crystalline Cinacalcet Hydrochloride or solutions originating from the synthesis or purification of Cinacalcet Hydrochloride.

Cinacalcet free base may be prepared by methods know in the literature, e.g. by reductive amination of 3-(3-trifluoromethyl-phenyl)-propionadehyde with 1 (R) - (1-naphthylethylamine as disclosed e.g. in Drugs of the future 2002, 27(99), 831-836. Alternatively Cinacalcet hydrochloride or a salt of Cinacalcet with an organic acid or inorganic acid may be used as starting material. A solution of these salts may be used directly as starting material for hydrochloride formation described below or these salts may be converted to the free base, e.g. by means of neutralization of a solution of these salts with a suitable base.

The solution of Cincalcet hydrochloride may then be provided by mixing of Cinacalcet free base with a hydrochloride source, e.g. aqueous or gaseous HCl, e.g. in stoichiometric amounts or using an excess of the hydrochloride source, e.g. up to 5 equivalents of the hydrochloride source in a solvent or solvent mixture as described above. A preferred way to generate Cinacalcet hydrochloride is the use of a trialkylsilylchloride in combination with a protic solvent as hydrochloride source as described in detail in Co-pending European application EP06116134, herein incorporated by reference.

A very preferred process for the preparation of a solution of Cinacalcet hydrochloride comprising the steps of:
(a) dissolving the free base of Cinacalcet in a protic solvent, and
(b) adding a trialkylsilylchloride, preferably trimethylchlorosilane, in an amount of about one mole equivalent calculated based on Cinacalcet free base. The resulting solution, for example in ethanole, may be used as starting material for the below described processes for the preparation of stable amorphous Cinacalcet hydrochloride.

In one embodiment the process for the production of a stable amorphous form of Cinacalcet hydrochloride comprising the step of removing the solvent from a solution of Cinacalcet hydrochloride in an organic solvent or a mixture of organic solvents.

Removal of solvent may be effected by spray drying, lyophilization or distillation. Distillation preferably is performed in vacuo.

Preferred solvents include acetone, dichloromethane, dioxane, mixtures of dioxane with water or diethylether, dimethylsulfoxyde, ethylacetate, ethylmethylketone, tetrahydrofurane, methanole, 1-proanole, 2- propanole, 2-propanole in combination with heptane,water or diethylether, or formic acid.

Alternatively, it is preferred that the solvent or solvent mixture is selected from a ketone, ether, ester, halogenated hydrocarbon, alcohole, hydrocarbon, water, or dimethylsulfoxyde.

A preferred ketone is a C₃- C₈ ketone. A preferred ester is selected from a C₁-C₄ carboxylic acid C₁-C₄ alkylester. A preferred ether is selected from a C₂-C₆ dialkylether, tetrahydrofurane or dioxane. A preferred halogenated hydrocarbon is dichloromethane. A preferred alcohole is a C₁-C₄ alcohole. A preferred hydrocarbon is a C₅-C₈ hydrocarbon.

Optionally a pharmaceutically acceptable carrier may be present in the removal step. As pharmaceutically acceptable carriers any material described in Encyclopedia of Pharmaceutical Technology (Vol 3, Table Ion page 345) may be used and preferred carriers are macgrogels, succinic acid, urea, pectin, desoxycholic acid, galactomannan, urethane, methylcellulose, hydroxypropylcellulose, hydroxypropylmethylcellulosephthalate, polyethylenglycol, poloxamers, polyacrylates, polymethylacrylates, hydroxyalkylxanthine, dextrose, sucrose, polyvinylpyrrolidon, galactose, maltose, xylitol, cyclodextrin, mannitol, sorbitol, and in particular polyethylenglycol, e.g. PEG 6000, maltose, sucrose, HPMC (hydroxypropyl -methylcellulose) or HPMCP (hydroxypropyl methylcellulose phthalate).

In a preferred embodiment the process for the production of stable amorphous cinacalcet hydrochloride comprises the steps of a) dissolving Cinacalcet free base in a solvent selected from the list consisting of acetone, dichloromethane, dioxane, mixtures of dioxane with water or diethylether,dimethylsulfoxyde, ethylacetate, ethylmethylketone, tetrahydrofurane, methanole, 1-proanole,2- propanole, and 2-propanole in combination with heptane, water or diethylether to obtain a solution of Cinacalcet and b) adding a hydrochloride source to the Cinacalcet solution obtained from step a), for example aqueous or gaseous HCl, in an amount sufficient to form a solution of Cinacalcet Hydrochloride, for example an amount of the hydrochloride source generating an about stoichiometric amount of hydrochloric acid or an excess of hydrochloric acid, e.g. up to 5 equivalents of hydrochloric acid, and c) removing the solvent by spray drying, lyophilization or distillation.

The invention also relates to a preferred process for the preparation of a dispersion of stable amorphous cinacalcet hydrochloride in a matrix material which process comprises the steps of a) dissolving Cinacalcet free base in a solvent selected from the list consisting of acetone, dichloromethane,dioxane,mixtures of dioxane with water or diethylether, dimethylsulfoxyde, ethylacetate, ethylmethylketone, tetrahydrofurane, methanole, 1-proanole, 2- propanole, and 2-propanole in combination with heptane, water or diethylether to obtain a solution of Cinacalcet and b) adding a hydrochloride source to the Cinacalcet solution obtained from step a), for example aqueous or gaseous HCl, in an amount sufficient to form a solution of Cinacalcet Hydrochloride, for example an amount of the hydrochloride source generating an about stoichiometric amount of hydrochloric acid or an excess of hydrochloric acid, e.g. up to 5 equivalents of hydrochloric acid, c) adding a pharmaceutical acceptable carrier at any stage before step d), and d) removing the solvent by spray drying, lyophilization or distillation. Preferred carriers are as described above. Preferred solvents are also as described above.

The present invention also relates to a pharmaceutical composition comprising stable amorphous cinacalcet hydrochloride and a suitable excipient.

The present invention also relates to pharmaceutical compositions comprising a dispersion of stable amorphous cinacalcet hydrochloride in a matrix material.

Preferred pharmaceutical compositions of the invention are oral dosage forms such as tablets, capsules, powders for oral suspension, pills and granules. For example the stable amorphous Cinacalcet Hydrochloride of the invention can be formulated as tablets for oral administration comprising from 20mg to 300mg and in particular from 30mg to 120mg Cinacalcet Hydrochloride, and further comprising pre-gelatinized starch, microcrystalline cellulose, povidone, crospovidone, colloidal silicon dioxide and magnesium stearate, preferably in amounts equivalent to the marketed product Sensipar® as sold in the US on the priority date. Preferably the tablets are also coated with color, clear film coat and/or carnauba wax.

The invention further relates to a method of treating primary and secondary hyperparathyroidism in a mammal comprising using stable amorphous Cinacalcet Hydrochloride, in particular comprising an inert stable amorphous form of Cinacalcet Hydrochloride. The invention further relates to the use of stable amorphous Cinacalcet Hydrochloride in the preparation of a medicament for the treatment of hyperparathyroidism, in particular for the prevention of treatment of osteoporosis, increased risked of renal bone disease, soft-tissue calcifications and vascular disease associated with hyperparathyroidism

The stable amorphous form of Cinacalcet Hydrochloride as obtained according to example 1 was analyzed by X-ray powder diffraction diagrams. The X-ray diffraction pattern was obtained using a Siemens D-5000 diffractometer (Bruker AXS, Karlsruhe, D) equipped with a theta/theta goniometer, a CuKα radiation source, a Goebel mirror (Bruker AXS, Karlsruhe, D), a 0.15° soller slit collimator and a scintillation counter. The patterns were recorded at a tube voltage of 40 kV and a tube current of 35 mA, applying a scan rate of 0.005° 2θs-1 in the angular range of 2 to 40° 2θ..

### DESCRIPTION OF THE FIGURE

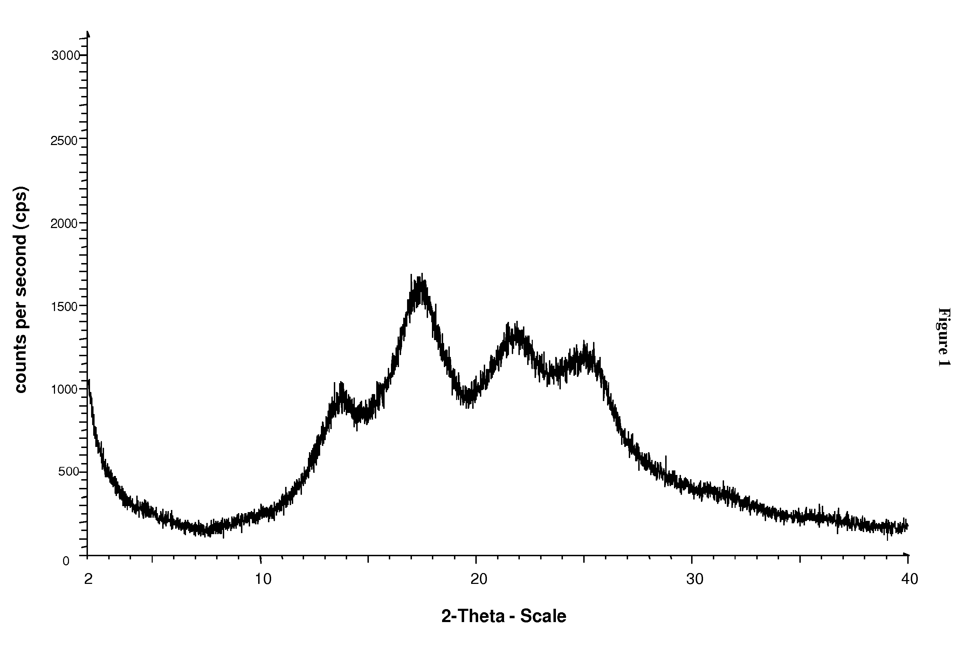

FIGURE 1: PXRD of amorphous Cinacalcet x HCl according to example 1

### EXAMPLES

The following examples describe the present invention in detail, but they are not to be construed to be in any way limiting for the present invention.

### Example I

50.2 mg Cinacalcet hydrochloride was dissolved in 2 ml of acetone at room temperature. After evaporating the solvent from a watch glass the amorphous form was obtained.

Yield: 50 mg.

In an analogy to example 1 amorphous Cinacalcet hydrochloride was prepared using the solvent or solvent mixtures described in table 1

**Table 1. Preparation of amorphous Cinacalcet**

| Solvent | Second solvent | Form |
|---|---|---|
| Acetone | | amorphous |
| Dichloromethane | | amorphous |
| Dioxane | + water | amorphous Amorphous |
| DMF | | Amorphous |
| DMSO | | Amorphous |
| Ethylacetate | | Amorphous |
| THF | | Amorphous |
| Methanol | | Amorphous |
| 1-Propanol | | Amorphous |
| 2-Propanol | + heptanes + water + ether | Amorphous Amorphous Amorphous Amorphous |

### Example 2

A sample of amorphous Cinacalcet Hydrochloride from Example 1 was stressed in a closed vial at 60°C for 24 hours. No decrease in assay was observed when measured by HPLC under the following conditions:
column: YMC-Pro C18 5 µm, 150x4.6mm; eluent: sulfamic acid/water, mobile phase A: 7.768g sulfamic acid in 2000g of water; mobile phase B: 7.768g sulfamic acid in 500g of water; measurement at a wavelength of 254 nm; inj. Vol 7 µm; temperature 40°C.
sample preparation: approximately 10 mg of sample dissolved in 25 ml of eluent B gradient:

| t(min) | %A | %B |
|---|---|---|
| 0 | 70 | 30 |
| 10 | 40 | 60 |
| 14 | 40 | 60 |
| 15 | 0 | 100 |
| 17 | 0 | 100 |
| 18 | 70 | 30 |

Area %/mg of starting material and stressed sample were compared

### Example 3

Stable amorphous Cinacalcet Hydrochloride from Example 1 was analyzed by XRPD. The obtained spectrum is shown in figure 1.

## Claims

1. Stable amorphous Cinacalcet Hydrochloride.

2. Process for the production of Cinacalcet Hydrochloride according to claim 1, comprising the step of removing the solvent from a solution of Cinacalcet hydrochloride in an organic solvent or a mixture of organic solvents.

3. Process according to claim 2 comprising the steps of a) dissolving Cinacalcet free base in an organic solvent to obtain a solution of Cinacalcet,
b) adding a hydrochloride source to the Cinacalcet solution obtained from step a) in an amount sufficient to form a solution of Cinacalcet Hydrochloride, and
c) removing the solvent by spray drying, lyophilization or distillation.

4. Process according to claims 2 or 3, wherein the organic solvent solvent is selected from the list consisting of acetone, dichloromethane, dioxane,mixtures of dioxane with water or diethylether, dimethylsulfoxyde, ethylacetate, ethylmethylketone, tetrahydrofurane, methanole, 1-proanole, 2-propanole, and 2-propanole in combination with heptane,water or diethylether, or formic acid.

5. Process according to claims 2 to 4, wherein a pharmaceutical acceptable carrier may be present in the step of removing the solvent.

6. Dispersion of stable amorphous cinacalcet hydrochloride in a matrix material

7. Process for the preparation of a dispersion of stable amorphous cinacalcet hydrochloride in a matrix material comprising the steps of
a) dissolving Cinacalcet free base in an organic solvent or mixture of organic solvents to obtain a solution of Cinacalcet and
b) adding a hydrochloride source to the Cinacalcet solution obtained from step a), in an amount sufficient to form a solution of Cinacalcet Hydrochloride,
c) adding a pharmaceutical acceptable carrier at any stage before step d), preferably after step b) and
d) removing the solvent, preferably by spray drying, lyophilization or distillation.

8. Pharmaceutical composition comprising stable amorphous cinacalcet hydrochloride and a suitable excipient..

9. Use of stable amorphous cinacalcet hydrochloride according to claim 1 for the preparation of a medicament, in particular for the treatment of primary and secondary hyperparathyroidism in a mammal, in particular in a human being.
